# EUROPEAN PATENT APPLICATION

(11) **EP 2 042 868 A1**
(43) Date of publication of application: **01.04.2009**
(21) Application number: 08014139.3
(22) Date of filing: 07.08.2008
(51) Int. Cl.: G01N 33/53, G01N 33/558

(54) **Immunochromatographic test device**

(30) Priority: 28.09.2007 JP 2007255309
(71) Applicant: SYSMEX CORPORATION, Kobe-shi, Hyogo 651-0073 (JP)
(72) Inventor: Hasegawa, Takehiro, Kobe-shi Hyogo 651-0073 (JP); Murakami, Masumi, Kobe-shi Hyogo 651-0073 (JP); Imoarai, Takeshi, Kobe-shi Hyogo 651-0073 (JP)
(74) Representative: HOFFMANN EITLE

(57) **Abstract**

The invention provides an immunochromatographic test device for detecting a test substance in a sample, comprising a chromatographic membrane carrier; a sample developing member; and a labeling substance holding member; wherein a substance being capable of reacting to a human anti-mouse antibody is held in a member disposed at a position which is more upstream on the basis of the sample developing direction than the chromatographic membrane carrier, as well as an a method for detecting a test substance in a sample by using an immunochromatographic test device andamethod for manufacturing an immunochromatographic test device.

## Description

### FIELD OF THE INVENTION

The present invention relates to an immunochromatographic test device, a method for detecting a test substance by using the immunochromatographic test device, and a method for manufacturing the immunochromatographic test device.

### BACKGROUND

As a test device for detecting a test substance in a sample by using a substance binding specifically to the test substance, an immunochromatographic test device is known. When a sample to be suspected of containing a test substance is added to a part (sample addition part) of this test device, the sample moves within the sample addition part by capillary phenomenon and reaches a labeling substance holding member in which a labeling substance capable of binding to the test substance is held. In the case where the test substance exists in the sample, the test substance binds to the labeling substance to form a test substance-labeling substance complex. When this complex moves in a chromatographic membrane carrier by further developing the sample by capillary phenomenon and reaches a detection zone on which a binding substance capable of binding to the test substance is immobilized, this complex binds to the binding substance. A labeling substance-test substance-binding substance complex in the form of a sandwich is thereby formed on the detection zone.

When the labeling substance contains colored carrier particles (colored latex particles or colloidal metal particles etc.), the detection zone is colored by capturing the labeling substance in the detection zone. By observing this coloration in the detection zone, the test substance in the sample can be detected.

In a test using such an immunochromatographic test device, a false-positive reaction by a substance contained in a sample and influencing the immunochromatographic reaction may be problematic. For example, biological samples collected from humans may contain a human anti-mouse antibody (HAMA). In this case, HAMA reacts with a mouse-derived antibody used as the binding substance for example in the immunochromatographic test device. It follows that even if the sample does not contain a test substance, a false-positive result would be given as if the test substance existed in the sample.

US Patent publication Serial No. 2007/0059682 has described an immunochromatographic test device with an HAMA capturing part having an HAMA capturing substance immobilized on a chromatographic membrane carrier. This immunochromatographic test device improves the accuracy of results by capturing HAMA present in a sample, with the HAMA capturing part.

However, there are cases where the immunochromatographic test device described in the patent application supra cannot sufficiently capture HAMA. As a result, HAMA not captured may give erroneous detection results.

### SUMMARY

The scope of the present invention is defined solely by the appended claims, and is not affected to any degree by the statements within this summary.

An object of the present invention is to provide an immunochromatographic test device capable of giving more accurate detection results than those of conventional immunochromatographic test devices, by reducing the influence, on immunochromatographic reaction, of a human anti-mouse antibody (HAMA) that may be contained in a sample.

Another object of the present invention is to provide a method for detecting a test substance by using the immunochromatographic test device and a method for manufacturing the immunochromatographic test device.

Even if the human anti-mouse antibody (HAMA) is contained in a sample, the influence of HAMA on immunochromatographic reaction can be sufficiently reduced by using the immunochromatographic test device of the present invention. Accordingly, a test substance in the sample can be accurately detected. It follows that when infection with pathogens such as viruses or microorganisms is examined, the possibility of false-positive results can be reduced. Therefore, more accurate examination results can be given.

### BRIEF DESCRIPTION OF THE DRAWINGS

Figs. 1A to 1D show embodiments of the immunochromatographic test device of the present invention; and
Fig. 2 shows a plan view of a chromatographic membrane carrier in Comparative Example.

### DETAILED DESCRIPTION OF THE EMBODIMENT

The immunochromatographic test device of the present invention is used to detect a test substance composed of an antigen or antibody in a sample.

The sample includes biological samples collected from humans and samples obtained by pretreatment of the biological samples. The biological samples collected from humans include blood, serum, stools, urine, saliva, nasal discharge, swabs, sweat, and tears. The pretreatment involves, for example, dissolvingabiological sample ina suitablepretreatment reagent and then filtering the resulting solution.

The test substance is not particularly limited as long as an antibody against the test substance can be obtained. Examples of the test substance include cells of bacteria, protists, fungi, and the like as well as viruses, proteins and polysaccharides. Specific examples of the test substance includeviruses suchas influenzaviruses (forexample, influenza AandBviruses), parainfluenzaviruses, RSviruses, rotaviruses, caliciviruses, coronaviruses, adenoviruses, enteroviruses, herpesviruses, human immunodeficiency viruses, hepatitis viruses, and disease viruses (coronaviruses) of severe acute respiratory syndrome; cells of Mycoplasma pneumoniae, Escherichia coli, Staphylococcus aureus, Streptococcus pneumoniae, Streptococcus piyogenes, and malaria parasite; causal organisms of various diseases suchas alimentary diseases, central nervous system diseases, and hemorrhagic fever, and metabolic products thereof; tumor markers such as carcinoembryonic antigens and Cyfra; and hormones. One or more of these test substances may be contained in a biological sample.

Particularly the immunochromatographic test device of the present invention can be preferably used in detection of one or more viruses selected from influenza viruses, coronaviruses, rotaviruses and adenoviruses.

In the immunochromatographic test device of the present invention, a substance (HAMA-reactive substance) capable of reacting with a human anti-mouse antibody (HAMA) is held in a member disposed at a position which is more upstream on the basis of a sample developing direction than a chromatographic membrane carrier. The HAMA-reactive substance may be held in one or more member (s) as long as the member (s) is/are disposed at a position which is more upstream on the basis of a sample developing direction than the chromatographic membrane carrier. The HAMA-reactive substance is not preferably immobilized in the member(s). That is, the HAMA-reactive substance is held so as to be developed together with a sample.

HAMA is a human antibody specifically recognizing a mouse immunoglobulin (mouse antibody). HAMA is often possessed by humans who have been exposed to a mouse antibody or humans who have undergone treatment with a pharmaceutical preparation using a mouse monoclonal antibody. In this specification, HAMA is not particularly limited as long as it is an antibody possessed by humans and capable of reacting with a mouse antibody. For example, HAMA may be a human-derived antibody capable of cross-reacting with another mammalian antibody such as a rabbit antibody or canine antibody.

The HAMA-reactive substance is not particularly limited as long as it is a substance capable of reacting with HAMA. Examples of the HAMA-reactive substance include an HAMA-reactive mouse antibody, its fragments (for example, Fc fragment, F(ab')2 fragment, Fab fragment and Fab' fragment). More specific examples of the HAMA-reactive substance include mouse-derived monoclonal antibodies produced by a hybridoma under Accession No. NITE BP-420 (Microbial Identification: HB2-2350) or a hybridoma under Accession No. NITE BP-421 (Microbial Identification: HB3-354), both of which have been deposited on September 25, 2007, with NITE Patent Microorganisms Depositary, National Institute of Technology and Evaluation; or "Unspecific Remover for Monoclonal Antibody" (produced by BioLink Technologies International).

The HAMA-reactive substances may be used singly or as a mixture of two or more thereof. Particularly, the HAMA-reactive substance is preferably one by mixing mouse-derived monoclonal antibodies produced by the hybridomas deposited under Accession Nos. NITE BP-420 and NITE BP-421 mentioned above.

In the immunochromatographic test device of the present invention, the HAMA-reactive substance is held in a member disposed at a position which is more upstream on the basis of a sample developing direction than a chromatographic membrane carrier.

Examples of the member disposed at a position which is more upstream on the basis of a sample developing direction than a chromatographic membrane carrier include a sample developing member and a labeling substance holding member. The member may be a second sample developing member different from the sample developing member or the labeling substance holding member.

According to the present invention, by holding the HAMA-reactive substance in the member disposed at a position which is more upstream on the basis of a sample developing direction than a chromatographic membrane carrier, the HAMA-reactive substance can be held in a larger amount than by immobilization of the HAMA-reactive substance onto a chromatographic membrane carrier. In addition, the reaction time of the HAMA-reactive substance with HAMA present in a sample can be longer than by immobilization of the HAMA-reactive substance onto a chromatographic membrane carrier. Accordingly, the influence of HAMA on the immunochromatographic reaction can be reduced.

In this specification, the terms "upstream on the basis of a sample developing direction" and "downstream on the basis of a sample developing direction" refer to relative directions assuming that the sample addition side is an upstream side and the sample developing (running) side is a downstream side.

In this specification, "the sample developing member supported on a substrate at a position which is more upstream on the basis of a sample developing direction than a chromatographic membrane carrier" means that at least one part of the sample developing member is disposed at any position which is more upstream on the basis of a sample developing direction, including the edge which is more upstream on the basis of a sample developing direction than a chromatographic membrane carrier.

The sample developing member in the immunochromatographic test device of the present invention has a sample addition part on which a sample is added, and is supported on a substrate at a position which is more upstream on the basis of a sample developing direction than a chromatographic membrane carrier. A sample developing member made of a material such as rayon, glass fibers or cellulose fibers can be used. The sample addition part, that is, a part on which a sample is added is preferably located at a part which is more upstream on the basis of a sample developing direction than the sample developing member.

The labeling substance holding member in the immunochromatographic test device of the present invention holds a labeling substance capable of binding to a test substance. The labeling substance holdingmember is supported on a substrate at a position which is more downstream on the basis of a sample developing direction than the sample addition part and which is more upstream on the basis of a sample developing direction than the chromatographic membrane carrier. A labeling substance holding member made of a material such as glass fibers, cellulose fibers, or plastic (for example, polyester, polypropylene or polyethylene) fibers can be used. The labeling substance holding member is preferably disposed so as to contact with the sample developing member.

The labeling substance capable of binding to a test substance is preferably one in which an antibody or antigen capable of binding to a test substance is labeled. Examples of a labeling component for labeling this antigen or antibody include particles obtained by labeling, with pigment molecules, fluorescent molecules, magnetic particles etc., agar, agarose, crosslinked agarose, crosslinked alginic acid, crosslinked guar gum, cellulose esters such as nitrocellulose and carboxy cellulose, gelatin, crosslinked gelatin, natural or synthetic resins such as latex, rubber, polyethylene, polypropylene, polystyrene, styrene-butadiene copolymers, polyvinyl chloride, polyvinyl acetate, polyacrylamide, polymethacrylate, styrene-methacrylate copolymers, polyglycidyl methacrylate and acrolein-ethyleneglycol dimethacrylate copolymers, and derivatives thereof, particles comprising inorganic materials such as glass (for example, activated glass), silica gel, kaolin, talc, silica-alumina, alumina, or barium sulfate; erythrocytes; and metal colloids such as gold colloids. Among them, latex particles having pigment molecules, fluorescent molecules or magnetic particles, and gold colloids, are preferable.

Two or more labeling substances capable of binding to the test substance may be used depending on the type of the test substance.

Preferably, the labeling substance holding member further holds a control labeling substance to confirm whether the sample has been suitably developed or not. The control labeling substance is not preferably substantially reactive to components in the sample. The control labeling substance can be used in combination with the following substance capable of binding to the control labeling substance. That is, the control labeling substance and the substance capable of binding to the control labeling substance are a combination of two substances capable of specifically binding to each other. Either of them may be used as the control labeling substance. The combination of two substances capable of specificallybinding to eachother includes a combination of a hapten and a protein capable of binding thereto, for example, a combination of streptavidin or avidin and biotin and a combination of 2 , 4-dinitrophenol or digoxin and a protein (for example, albumin, casein, fibrinogen or the like).

A label for the control labeling substance may be the same label as in the above labeling substance. The control labeling substance may be labeled with the same labeling component as in the labeling substance or may be labeled with a different labeling component.

A substrate for the immunochromatographic test device of the present invention is not particularly limited as long as it is used as a substrate for a normal immunochromatographic test device. For example, plastics, paper or glass may be used. The substrate preferably has a sticky surface to dispose the chromatographic membrane carrier, the sample developing member, the labeling substance holding member etc. thereon.

The chromatographic membrane carrier in the immunochromatographic test device of the present invention has a detection part on which the binding substance capable of binding to a test substance is immobilized. The chromatographic membrane carrier may be made of a material capable of binding to a protein via physical action such as electrostatic action or hydrophobic interaction and simultaneously developing the sample by capillary phenomenon. Examples of such material include nitrocellulose, nylon (for example, a modified nylon into which an amino group optionally having a carboxyl or alkyl group as a substituent is introduced), polyvinylidene difluoride (PVDF), and cellulose acetates.

The binding substance to be immobilized on the detection part of the chromatographic membrane carrier maybe any substance capable of binding to a test substance. The binding substance is particularly preferably an antibody or antigen capable of specifically binding to a test substance. The binding substance is more preferably one which binds to a test substance at a site that is different from the site where the labeling substance binds to the test substance.

The chromatographic membrane carrier may have only one detection zone or two or more detection zones, depending on the type of the test substance. When the chromatographic membrane carrier has two or more detection zones, different kinds of binding substances capable of binding to the test substance are immobilized in the detection zones, respectively.

The chromatographic membrane carrier preferably has a control zone on which a substance capable of binding to the control labeling substance is immobilized in order to confirm whether the sample has been suitably developed or not.

The detection zone and the control zone may be in the form of a line, a circle, a square or the like.

The immunochromatographic test device of the present invention can be provided with an absorption member in addition to the chromatographic membrane carrier, the sample developing member, and the labeling substance holding member. The absorption member may be made of a material such as cellulose or glass fibers. The absorption member is preferably disposed so as to contact with the chromatographic membrane carrier at a position which is more downstream on the basis of a sample developing direction than the chromatographic membrane carrier. By arranging the absorption member at such a position, the test device can increase the development speed of a sample.

Using a conventionally known method for manufacturing an immunochromatographic test device, the immunochromatographic test device of the present invention can be manufactured by allowing an HAMA-reactive substance to be held on a member disposed at a position which is more upstream on the basis of a sample developing direction than the chromatographic membrane carrier.

In the conventional production method, the labeling substance holding member that holds a labeling substance, for example, is prepared by using a labeling substance-containing buffer solution. More specifically, a labeling substance-containing buffer solution is contained in the labeling substance holding member by impregnating the labeling substance holding member with the buffer or by adding the buffer solution to the labeling substance holding member. Then, the labeling substance holding member containing the buffer solution can be dried to prepare the labeling substance holding member that holds a labeling substance. In this case, an HAMA-reactive substance can be also added to the buffer solution to hold not only the labeling substance but also the HAMA-reactive substance on the labeling substance holding member. By adding a control labeling substance to the buffer solution, the control labeling substance can be also held on the labeling substance holding member.

The sample developing member that holds an HAMA-reactive substance can be also prepared by using a buffer solution containing an HAMA-reactive substance. That is, the sample developing member that holds an HAMA-reactive substance can be prepared by impregnating the sample developing member with the buffer solution or adding the buffer solution to the sample developing member, followed by drying. A second sample developing member that holds an HAMA-reactive substance can be also prepared in the same manner by using a buffer solution containing an HAMA-reactive substance.

The labeling substance holding member, the sample developing member or the second sample developingmemberprepared as described above can be assembled together with other components prepared by methods known in the art, for example, a substrate, a chromatographic membrane carrier, a labeling substance holding member or a sample developing member, an arbitrary second sample developing member and an arbitrary absorption member, and then cut to manufacture the immunochromatographic test device of the present invention.

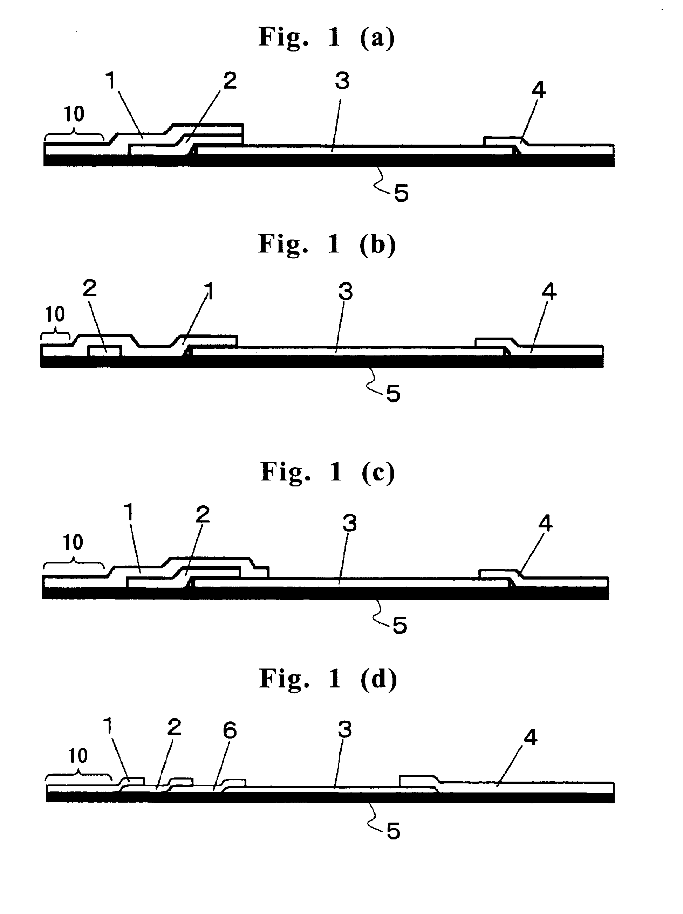

Embodiments of the immunochromatographic test device of the present invention are shown in Fig. 1A, Fig. 1B, Fig. 1C and Fig. 1D. The immunochromatographic test devices shown in Fig. 1A, Fig. 1B and Fig. 1C have a sample developing member 1, a labeling substance holding member 2, a chromatographic membrane carrier 3, an absorption member 4 and a substrate 5. The immunochromatographic test device shown in Fig. 1D has a sample developing member 1, a labeling substance holding member 2, a chromatographic membrane carrier 3, an absorption member 4, a substrate 5 and a second sample developing member 6. A sample addition part 10 is located at a more upstream part on the basis of the sample developing direction than the sample developing member 1.

In a preferable aspect of the immunochromatographic test device of the present invention, the labeling substance holding member 2 is disposed so as to contact with the sample developing member 1 and the chromatographic membrane carrier 3. This aspect is shown in, for example, Fig. 1A and Fig. 1C.

In another preferable aspect of the immunochromatographic test device of the present invention, the labeling substance holding member 2 is disposed so as to leave space from the chromatographic membrane carrier 3. This aspect is shown in, for example, Fig. 1B and Fig. 1D. The space can be suitably selected depending on the size of the immunochromatographic device and the type of a test substance to be detected.

In still another preferable aspect of the immunochromatographic test device of the present invention, the sample developing member 1 is disposed so as to contact with the chromatographic membrane carrier 3. This aspect is shown in, for example, Fig. 1B and Fig. 1C.

Preferably, the immunochromatographic test device of the present invention is further provided with a second sample developing member 6 supported on the substrate 5 at a position which is more downstream on the basis of the sample developing direction than the sample addition part 10, and which is more upstream on the basis of the sample developing direction than the chromatographic membrane carrier 3. The second sample developing member 6 may be disposed at a position which is either more upstream or downstream on the basis of the sample developing direction than the labeling substance holding member 2. There may be also two or more second sample developing members 6.

When the immunochromatographic test device of the present invention is provided with the second sample developing member 6, at least one member selected from the sample developing member 1, the second sample developing member 6 and the labeling substance holding member 2 may hold the HAMA-reactive substance. The second sample developing member 6 may be made of the same material as in the sample developing member 1.

### EXAMPLES

### Example 1

Mouse-derived monoclonal antibodies produced by hybridomas deposited under Accession Nos. NITE BP-420 and NITE BP-421, with NITE Patent Microorganisms Depositary, National Institute of Technology and Evaluation, were held as HAMA-reactive substances on a labeling substance holding member of a commercial influenza virus detection immunochromatographic test device (Poctem influenza A/B, Sysmex Corporation) to prepare an immunochromatographic test device of the present invention.

That is, a labeling substance holding member of Poctem influenza A/B was prepared in the following manner.

1050 µg of blue colored polystyrene latex particles (particle size 0.3 µm) sensitizing an anti-influenza A virus monoclonal antibody were suspended in 3950 µL of a dispersing buffer solution (phosphate buffer solution, pH 7.0, containing BSA and sucrose) to prepare anti-influenza A virus monoclonal antibody-sensitized latex particles.

1050 µg of blue colored polystyrene latex particles (particle size 0.3 µm) sensitizing an anti-influenza B virus monoclonal antibody were suspended in 3950 µL of a dispersing buffer solution (phosphate buffer solution, pH 7.0, containing BSA and sucrose) to prepare anti-influenza B virus monoclonal antibody-sensitized latex particles.

1150 µg of red polystyrene latex particles (particle size 0.19 µm) sensitizing streptavidin were suspended in 3850 µL of a dispersing buffer solution (phosphate buffer solution, pH 7. 0, containing BSA and sucrose) to prepare streptavidin-sensitized latex particles.

The mouse-derived monoclonal antibodies produced by the two types of hybridomas described above were dissolved at a concentration of 1.75 mg/mL in 1 mL of an HAMA-reactive substance buffer solution (phosphate buffer solution, pH 7.0) to prepare HAMA-reactive substance solutions, respectively.

3600 µL of the anti-influenza A virus monoclonal antibody-sensitized latex particles, 3600 µL of the anti-influenza B virus monoclonal antibody-sensitized latex particles, 200 µL of the streptavidin-sensitized latex particles, and 800 µL of the HAMA-reactive substance solution, each of which was prepared as described above, were mixed to give 8200 µL of a mixed latex containing the HAMA-reactive substances.

832 µL of the resulting mixed latex containing the HAMA-reactive substances was added to a glass fiber pad (300 mm × 5 mm × 0.28 mm) and dried in a vacuum drier to prepare a labeling substance holding member.

The labeling substance holding member thus prepared was assembled with a substrate, a chromatographic membrane carrier and a sample developing member from Poctem influenza A/B, and then cut into immunochromatographic test devices of the present invention.

This immunochromatographic test device held 10 µg of the HAMA-reactive substances per one test strip.

Poctem influenza A/B is a test device having the structure shown in Fig. 1A, wherein binding substances capable of binding to a test substance, that is, an anti-influenza A virus mouse monoclonal antibody and an anti-influenza B virus mouse monoclonal antibody, and a substance capable of binding to a control labeling substance, that is, biotin-BSA, are immobilized in detection zones and a control zone respectively on a chromatographic membrane carrier.

### Comparative Example 1

Poctem influenza A/B was used in Comparative Example 1.

### Comparative Example 2

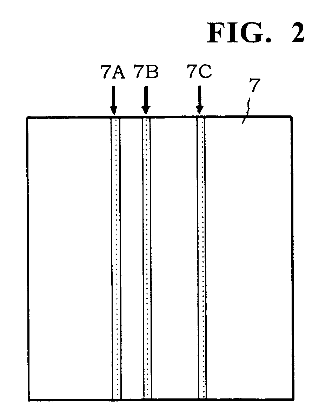

Poctem influenza A/B having the same HAMA-reactive substances as in Example 1 immobilized on a first detection zone 7A (see Fig. 2) of a chromatographic membrane carrier of Poctem influenza A/B was used in Comparative Example 2.

Hereinafter, a method for preparing the chromatographic membrane carrier in Comparative Example 2 is described with reference to Fig. 2.

Fig. 2 is a plan view of the chromatographic membrane carrier. As shown in Fig. 2, the chromatographic membrane carrier 7 composed of a nitrocellulose membrane has a first detection zone 7A, a second detection zone 7B and a control zone 7C.

The chromatographic membrane carrier in Comparative Example 2 was coated using an antibody applicator (BioDot Inc.) with the following solutions in the first detection zone 7A, the second detection zone 7B and the control zone 7C.

### First detection zone 7A:

A solution of the above-mentioned two types of mouse monoclonal antibodies contained as HAMA-reactive substances diluted at 1.75 mg/ml respectively with a phosphate buffer solution, pH 7.0.

### Second detection zone 7B:

A solution of an anti-influenza B virus monoclonal antibody diluted at a concentration of 1.5 mg/mL with a phosphate buffer solution, pH 7.0.

### Control zone 7C:

A solution of biotin-BSA diluted at a concentration of 2.0 mg/mL with a phosphate buffer solution, pH 7.0.

The chromatographic membrane carriers in Example 1 and Comparative Example 1 have been coated with a solution of an anti-influenza A virus monoclonal antibody diluted at a concentration of 2.0 mg/mL with a phosphate buffer solution, pH 7.0, in the first judgment part 7A thereof.

The chromatographic membrane carriers coated with the respective solutions were dried at 50°C for 30 minutes.

After drying, the chromatographic membrane carrier 7 was blocked by dipping into a blocking solution (BSA-containing phosphate buffer solution, pH 7.0). After blocking, the chromatographic membrane carrier 7 was washed with a cleaning fluid (SDS-containing phosphate buffer solution, pH 7.0) and then dried at 40°C for 120 minutes to prepare the chromatographic membrane carrier in Comparative Example 2.

The resulting chromatographic membrane carrier in Comparative Example 2 was assembled with a substrate, a labeling substance holding member and a sample developing member from Poctem influenza A/B, and then cut into immunochromatographic test devices in Comparative Example 2.

On the chromatographic membrane carrier of the immunochromatographic test device obtained in Comparative Example 2, the HAMA-reactive substances were immobilized in an amount of 4.1 µg per one test strip. When the HAMA-reactive substances larger than this amount were to be immobilized, the HAMA-reactive substances were found to be precipitated on the surface of the chromatographic membrane carrier. Accordingly, the amount of the HAMA-reactive substances that can be immobilized on this chromatographic membrane carrier is considered to be significantly smaller than the amount of the HAMA-reactive substances held on the immunochromatographic test device in Example 1.

### Example 1

Anti-mouse IgG (MBL303G Anti-Mouse IgG (Goat), Lot No. 130) was diluted at concentrations shown in Table 1 with a dilution buffer shown below. 200 µL of each of the resulting samples was added to the sample addition part of the immunochromatographic test device in each of Example 1 and Comparative Examples 1 and 2. After 15 minutes, the coloration density of the detection zone was visually determined.

This determination was conducted by preparing visual observation determination specimens in which 1+ is given to a line intensity of equal to or more than 0. 015 and less than 0. 03 measured by TSR 3000 membrane strip reader manufactured by BioDot Inc., 2+ is given to a line intensity of equal to or more than 0.03 and less than 0.08, and 3+ is given to a line intensity of equal to ormore than 0.08, and then comparing the line intensity on each test device with the visual observation determination specimen. A line with lower color intensity than the visual observation determination specimen of 1+ was determined to be +w.

A dilution buffer not containing the anti-mouse IgG was used as the negative control (NC).

A composition of the dilution buffer is as follows:

### <Dilution buffer>

| | |
|---|---|
| Monobasic sodium phosphate·2H₂O | 78 g |
| Sodium chloride | 45 g |
| Sodium azide | 2.5 g |
| Bovine albumin | 50 g |
| 4 N sodium hydroxide | 87.5 mL |
| Ultrapure water | 5 L |

| | |
|---|---|
| * Adjusted to pH 7.0 with 1 N sodium hydroxide and 1 Nhydrochloric acid | |

The results are shown in Table 1. The column "A" shows determination results in the first detection zone on which the anti-influenza A virus monoclonal antibody has been immobilized. The column "B" shows determination results in the second detection zone on which the anti-influenza B virus monoclonal antibody has been immobilized. "-" indicates that the coloration of the detection zones could not be visually confirmed.

In Comparative Example 2, the HAMA-reactive substances were immobilized on the first detection zone as described above. Accordingly, judgment results in the second detection zone, that is, the column "B" only is indicated in Comparative Example 2.

Though not shown in the table, the coloration of the control zone was confirmed in every experiment. That is, it was confirmed that the sample had been suitably developed.

**Table 1**

| Concentration of anti-mouse IgG | Comparative Example 1 | | Example 1 | | Comparative Example 2 |
|---|---|---|---|---|---|
| | A | B | A | B | B |
| 168µg/mL | 3+ | 3+ | 1+ | 2+ | 3+ |
| 16.8µg/mL | 2+ | 2+ | - | - | 2+ |
| 1.68µg/mL | +w | +w | - | - | +w |
| NC | - | - | - | - | - |

From the results in Table 1, it can be seen that although no influenza virus is contained in the sample, the detection zones (first and second detection zones) in Poctem influenza A/B (Comparative Example 1) show a false-positive result due to the presence of the anti-mouse IgG.

This false-positive result could not be avoided even by using the immunochromatographic test device in Comparative Example 2 where the HAMA-reactive substances were immobilized on the chromatographic membrane carrier.

The immunochromatographic test device of the present invention in Example 1 showed a slightly false-positive result at an anti-mouse IgG concentration of 168 µg/mL, but can be seen to provide more accurate results than by the test devices in Comparative Examples 1 and 2.

### Example 2

The immunochromatographic test device of the present invention was prepared in the same manner as in Example 1 except that in place of Poctem influenza A/B in Example 1, the same HAMA-reactive substances as used in Example 1 were held on a labeling substance holding member of Poctem S influenza (Sysmex Corporation).

Poctem S influenza, similar to Poctem influenza A/B, has the structure shown in Fig. 1B, wherein binding substances capable of binding to a test substance, that is, ananti-influenza A virus mouse monoclonal antibody and an anti-influenza B virus mouse monoclonal antibody, and a substance capable of binding to a control labeling substance, that is, biotin-BSA, are immobilized in detection zones and a control zone respectively on a chromatographic membrane carrier.

### Comparative Example 3

Poctem S influenza only was used in Comparative Example 3.

### Experimental example 2

The immunochromatographic test devices in Examples 1 and 2 and Comparative Examples 1 and 3 were used to examine samples collected from patients.

According to instructions attached to Poctem influenza A/B or Poctem S influenza, biological samples (nasal discharge, nasal swab and throat swab) collected from patients were treated with an attached sample extracting reagent to prepare samples.

About 200 µL of the resulting sample was added to the sample addition part of the immunochromatographic test device in each of Examples 1 and 2 and Comparative Examples 1 and 3, and the line intensity of the detection zone was determined in the same manner as in Example 1. A line intensity of +w or more was determined to be influenza virus-positive.

In a control experiment, the same biological sample was detected for its influenza virus antigen by RT-PCR (reverse transcriptase-polymerase chain reaction).

The number of samples determined to be positive by the immunochromatographic test device or by RT-PCR is shown in "A" (positive for influenza A virus), "B" (positive for influenza B virus) or "A/B" (positive for both influenza A and B viruses) in Table 2. The column "-" in Table 2 shows the number of samples determined to be negative.

**Table 2**

| (A) Immunochromatographic test device in Comparative Example 1 | | | | | | |
|---|---|---|---|---|---|---|
| | | RT-PCR | | | | Total |
| | | A | B | A/B | - | |
| Comparative Example 1 | A | 80 | 0 | 0 | 0 | 80 |
| | B | 0 | 62 | 0 | 0 | 62 |
| | A/B | 0 | 0 | 1 | 1 | 2 |
| | - | 22 | 21 | 0 | 141 | 184 |
| Total | | 102 | 83 | 1 | 142 | 328 |

| (B) Immunochromatographic test device in Example 1 | | | | | | |
|---|---|---|---|---|---|---|
| | | RT-PCR | | | | Total |
| | | A | B | A/B | - | |
| Example 1 | A | 77 | 0 | 0 | 0 | 77 |
| | B | 0 | 62 | 0 | 0 | 62 |
| | A/B | 0 | 0 | 1 | 0 | 1 |
| | - | 25 | 21 | 0 | 142 | 188 |
| Total | | 102 | 83 | 1 | 142 | 328 |

| (C) Immunochromatographic test device in Comparative Example 3 | | | | | | |
|---|---|---|---|---|---|---|
| | | RT-PCR | | | | Total |
| | | A | B | A/B | - | |
| Comparative Example 3 | A | 104 | 0 | 0 | 2 | 106 |
| | B | 0 | 57 | 0 | 0 | 57 |
| | A/B | 0 | 0 | 0 | 3 | 3 |
| | - | 33 | 22 | 0 | 132 | 187 |
| Total | | 137 | 79 | 0 | 137 | 353 |

| (D) Immunochromatographic test device in Example 2 | | | | | | |
|---|---|---|---|---|---|---|
| | | RT-PCR | | | | Total |
| | | A | B | A/B | - | |
| Example 2 | A | 104 | 0 | 0 | 0 | 104 |
| | B | 0 | 54 | 0 | 0 | 54 |
| | A/B | 0 | 0 | 0 | 0 | 0 |
| | - | 33 | 25 | 0 | 137 | 195 |
| Total | | 137 | 79 | 0 | 137 | 353 |

As can be seen from the results in Table 2, there are samples (shaded columns in Table 2 (A), (C)) that are determined to be absent from influenza virus by RT-PCR but are determined to be positive by the conventional immunochromatographic test devices, but when the immunochromatographic test devices of the present invention are used, even such samples can be determined to be negative in accordance with the results of RT-PCR. That is, the possibility of false-positive results can be reduced by using the immunochromatographic test devices of the present invention.

### Example 3

The immunochromatographic test device of the present invention was prepared by using a commercial unspecific absorptive antibody (Unspecific Remover for Monoclonal Antibody, Lot 061002, produced by BioLink Technologies International) as an HAMA-reactive substance in place of the HAMA-reactive substances used in Example 2.

The amount of the HAMA-reactive substance held per one test strip was 72 µg.

### Experimental example 3

Using the immunochromatographic test devices in Example 3 and Comparative Example 3, line intensity was determined using HAMA serums (human anti-mouse antibody-containing serums) as samples.

The following HAMA serums were used. HAMA serum 1: BioReclamation (produced by Kokusai Bio) BRH22942 HAMA serum 2: BioReclamation (produced by Kokusai Bio) BRH40929 HAMA serum 3: BioReclamation (produced by Kokusai Bio) BRH40930 HAMA serum 4: BioReclamation (produced by Kokusai Bio) BRH40932

150 µl of the HAMA serum was mixed with 800 µL of a sample extracting reagent attached to Poctem S influenza. About 200 µL of the resulting sample was added to the sample addition part of each immunochromatographic test device, and after 10 minutes, its line intensity was determined in the same manner as in Example 1.

200 µL of the sample extracting reagent was used as the negative control (NC).

The results are shown in Table 3. "-" indicates that the coloration of the detection zone could not be visually confirmed. Though not shown in the table, the coloration of the control part was confirmed in every experiment. That is, it was confirmed that the sample had been suitably developed.

**Table 3**

| | Comparative Example 3 | | Example 3 | |
|---|---|---|---|---|
| | A | B | A | B |
| HAMA serum 1 | - | 2+ | - | 1+ |
| HAMA serum 2 | 1+ | - | - | - |
| HAMA serum 3 | 1+ | - | - | - |
| HAMA serum 4 | 1+ | 2+ | - | +w |
| N.C | - | - | - | - |

From the results in Table 3, it can be seen that the immunochromatographic test device of the present invention using the commercial unspecific absorptive antibody as the HAMA-reactive substance could reduce the possibility of false-positive results as compared with the conventional immunochromatographic test device.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. An immunochromatographic test device for detecting a test substance in a sample, comprising:
a chromatographic membrane carrier having a detection zone at which a binding substance being capable of binding to the test substance is immobilized;
a sample developing member disposed at a position which is more upstream on the basis of a sample developing direction than the chromatographic membrane carrier, and having a sample addition part on which the sample is added; and
a labeling substance holding member disposed at a position which is more downstream on the basis of the sample developing direction than the sample addition part and more upstream on the basis of the sample developing direction than the chromatographic membrane carrier, and holding a labeling substance being capable of binding to the test substance;
wherein a substance being capable of reacting to a human anti-mouse antibody is held in a member disposed at a position which is more upstream on the basis of the sample developing direction than the chromatographic membrane carrier.

2. The immunochromatographic test device according to claim 1, wherein the substance being capable of reacting to a human anti-mouse antibody is held in the labeling substance holding member.

3. The immunochromatographic test device according to claim 1 or 2, wherein the substance being capable of reacting to a human anti-mouse antibody is held in the sample developing member.

4. The immunochromatographic test device according to any one of claims 1 to 3, wherein the labeling substance holding member is disposed so as to contact with the sample developing member and the chromatographic membrane carrier.

5. The immunochromatographic test device according to any one of claims 1 to 4, wherein the labeling substance holding member is disposed so as to leave space from the chromatographic membrane carrier.

6. The immunochromatographic test device according to any one of claims 1 to 5, wherein the sample developing member is disposed so as to contact with the chromatographic membrane carrier.

7. The immunochromatographic test device according to any one of claims 1 to 6, wherein the sample developing member is disposed so as to leave space from the chromatographic membrane carrier.

8. The immunochromatographic test device according to any one of claims 1 to 7, wherein the substance being capable of reacting to the human anti-mouse antibody is an antibody being capable of reacting to a human anti-mouse antibody or a fragment of the antibody being capable of reacting to a human anti-mouse antibody.

9. The immunochromatographic test device according to claim 8, wherein the antibody being capable of reacting to a human anti-mouse antibody is produced by a hybridoma selected from at least one of an international deposit number NITE BP-420 and an international deposit number NITE BP-421.

10. The immunochromatographic test device according to any one of claims 1 to 9, wherein the labeling substance holding member further holds a control labeling substance, and
the chromatographic membrane carrier further has a control zone at which a substance being capable of binding to the control labeling substance is immobilized.

11. The immunochromatographic test device according to any one of claims 1 to 10 further comprises a second sample developing member disposed at a position which is more downstream on the basis of the sample developing direction than the sample addition part and more upstream on the basis of the sample developing direction than the chromatographic membrane carrier.

12. The immunochromatographic test device according to claim 11, wherein the substance capable of reacting with a human anti-mouse antibody is held in the second sample developing member.

13. The immunochromatographic test device according to any one of claims 1 to 12, wherein the substance being capable of reacting to the human anti-mouse antibody is held in the member so as to be developed with the sample.

14. A method for detecting a test substance in a sample by using an immunochromatographic test device comprising a chromatographic membrane carrier having a detection zone at which a binding substance being capable of binding to a test substance is immobilized, comprising steps of:
applying the sample to the immunochromatographic test device;
contacting a substance being capable of reacting to a human anti-mouse antibody and a labeling substance being capable of binding to the test substance with the sample in a member disposed at a position which is more upstream on a basis of the sample developing direction than the chromatographic membrane carrier;
contacting the binding substance being capable of binding to the test substance immobilized at the detection zone with the sample;
detecting the labeling substance bound to the detection zone through the test substance.

15. A method for manufacturing an immunochromatographic test device comprising a chromatographic membrane carrier having a detection zone at which a binding substance being capable of binding to a test substance is immobilized, for detecting the test substance in a sample, comprising a step of holding a substance being capable of reacting to a human anti-mouse antibody in a member disposed at a position which is more upstream on the basis of a sample developing direction than the chromatographic membrane carrier.
